# EUROPEAN PATENT APPLICATION

(11) **EP 4 080 460 A1**
(43) Date of publication of application: **26.10.2022**
(21) Application number: 22164511.2
(22) Date of filing: 25.03.2022
(51) Int. Cl.: G06T 11/00

(54) **MEDICAL IMAGE PROCESSING APPARATUS, MEDICAL IMAGE DIAGNOSIS APPARATUS, AND PROGRAM**

(30) Priority: 26.03.2021 JP 2021053318
(71) Applicant: Canon Medical Systems Corporation, Tochigi 324-0036 (JP)
(72) Inventor: TESHIGAWARA, Manabu, Tochigi (JP)
(74) Representative: Marks & Clerk LLP

(57) **Abstract**

A medical image processing apparatus (100) according to an embodiment is a medical image processing apparatus (100) that performs processing using a trained model to generate a first output medical image by subjecting a first input medical image to predetermined processing, and includes a processing circuit (150). The processing circuit (150) generates a plurality of second output medical images for a second input medical image by randomly switching ON/OFF a connection of a plurality of neurons included in the trained model.

## Description

### FIELD

Embodiments described herein relate generally to a medical image processing apparatus, a medical image diagnosis apparatus, a medical image processing method, and a program.

### BACKGROUND

When performing image processing on a medical image, it is desirable to have a means to verify credibility (confidence level) of the image processing itself. For example, when image filter processing is performed on a medical image, it is desirable to check whether characteristics of the image filter processing itself do not affect the diagnostic performance.

From this perspective, for example, when a neural network is applied to a medical image, there is a case of taking an approach that after generating a trained model by training with sufficient amount of training data, the trained model is applied to another test data to verify adequacy, general versatility, and accuracy of a result.

However, by such a method, the adequacy of a result after application of the neural network cannot be quantitatively evaluated for data other than the training data used at the time of generating the trained model and the test data used at the time of verification of the trained model.

### SUMMARY OF INVENTION

A medical image processing apparatus provided in one aspect of the present disclosure is a medical image processing apparatus configured to perform processing using a trained model to generate a first output medical image by subjecting a first input medical image to predetermined processing, comprising a processing circuit.

The processing circuit generates a second output medical image for a second input medical image by randomly switching ON/OFF a connection of a plurality of neurons included in the trained model.

The processing circuit may generate information relating to credibility of an output of the trained model based on the second output medical images.

The processing circuit may generate a third output medical image by combining the second output medical images.

The processing circuit may generate a fourth output medical image that is an image indicating a magnitude of variation of the second output medical images.

The processing circuit may display the third output medical image and the information on a display.

The processing circuit may accept specification of a region of interest from a user, and
may generate the information based on the region of interest.

The processing circuit may calculate a weight in superposition for each pixel based on the information, and may generate a composite medical image by superimposing the first input medical image and the first output medical image based on the weight.

A medical image processing apparatus provided in one aspect of the present disclosure is a medical image processing apparatus configured to perform processing using a plurality of trained models to generate a first output medical image by subjecting a first input medical image to predetermined processing, and includes a processing circuit configured to generate a second output medical image for a second input medical image based on the trained models.

A medical image diagnosis apparatus provided in one aspect of the present disclosure includes a medical image processing apparatus configured to perform processing using a trained model to generate a first output medical image by subjecting a first input medical image to predetermined processing, and
the medical image processing apparatus is configured to generate a plurality of second output medical images for a second input medical image by randomly switching ON/OFF a connection of a plurality of neurons included in the trained model.

A medical image processing method provided in one aspect of the present disclosure is a medical image processing method performed by a medical image processing apparatus configured to perform processing using a trained model to generate a first output medical image by subjecting a first input medical image to predetermined processing, and
a plurality of second output medical images are generated for a second input medical image by randomly switching ON/OFF a connection of a plurality of neurons included in the trained model.

A non-transitory computer-readable recording medium provided in one aspect of the present disclosure stores a program that causes a computer performing processing using a trained model to generate a first output medical image by subjecting a first input medical image to predetermined processing, to execute processing of generating a plurality of second output medical images for a second input medical image by randomly switching ON/OFF a connection of a plurality of neurons included in the trained model.

### BRIEF DESCRIPTION OF THE DRAWINGS

FIG. 1 is a diagram illustrating an example of a medical image processing apparatus according to an embodiment;
FIG. 2 is a diagram illustrating an example of a medical diagnostic imaging apparatus according to the embodiment;
FIG. 3 is a diagram illustrating an example of a neural network according to the embodiment;
FIG. 4 is a diagram illustrating an example of a neural network according to the embodiment;
FIG. 5 is a diagram illustrating an example of a neural network according to the embodiment;
FIG. 6 is a flowchart illustrating a flow of processing performed by the medical image processing apparatus according to the embodiment;
FIG. 7 is a flowchart illustrating a flow of processing performed by the medical image processing apparatus according to the embodiment;
FIG. 8 is a diagram explaining processing performed by the medical image processing apparatus according to the embodiment;
FIG. 9 is a diagram illustrating an example of an image output by the medical image processing apparatus according to the embodiment; and
FIG. 10 is a diagram explaining an example of an image output by the medical image processing apparatus according to the embodiment.

### DETAILED DESCRIPTION

A medical image processing apparatus provided according to one aspect of the present embodiment performs processing using a trained model to generate a first output medical image by subjecting a first input medical image to predetermined processing, the apparatus comprising a processing circuit. The processing circuit generates a plurality of second output medical images for a second input medical image by randomly switching ON/OFF a connection of a plurality of neurons included in the trained model.

Hereinafter, embodiments of a medical image processing apparatus, a medical image diagnosis apparatus, a medical image processing method, and a non-transitory computer-readable storage medium will be explained in detail with reference to the drawings.

### First Embodiment

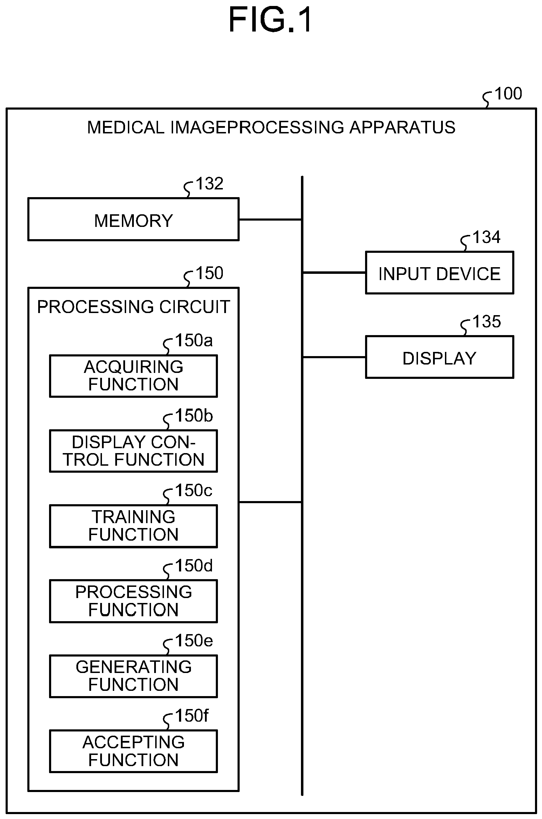

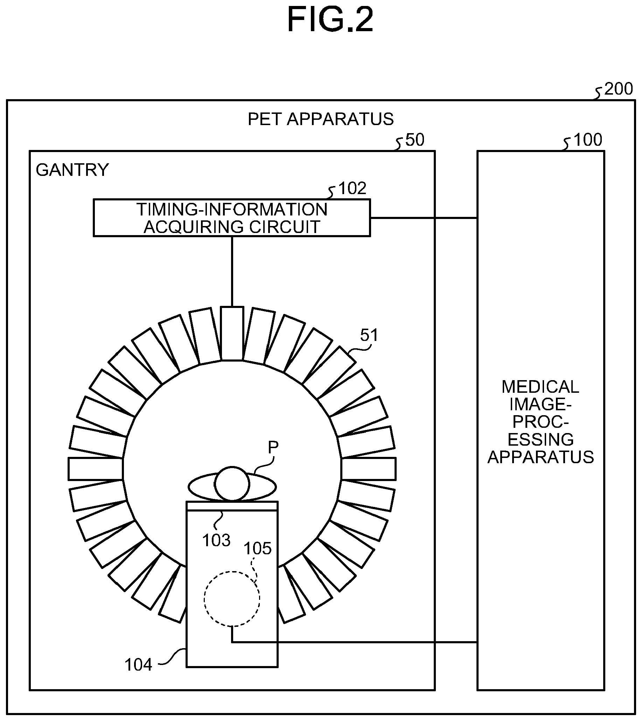

First, a configuration example of the medical image processing apparatus and the medical image diagnosis apparatus according to an embodiment will be explained by using FIG. 1 and FIG. 2. FIG. 1 is a diagram illustrating a medical image processing apparatus 100 according to the embodiment. Moreover, FIG. 2 is a diagram illustrating an example of a medical image diagnosis apparatus equipped with the medical image processing apparatus 100 according to the embodiment. In FIG. 2, a case in which the medical image diagnosis apparatus equipped with the medical image processing apparatus 100 is a PET apparatus 200 is explained. However, embodiments are not limited to the case in which the medical image diagnosis apparatus is the PET apparatus 200, but the medical image diagnosis apparatus may be other medical image diagnostic apparatuses, for example, an ultrasound diagnostic apparatus, a magnetic resonance imaging apparatus, an X-ray CT apparatus, and the like. Furthermore, the medical image processing apparatus may function independently as a medical image processing apparatus without being equipped in a medical image diagnosis apparatus.

In FIG. 1, the medical image processing apparatus 100 includes a memory 132, an input device 134, a display 135, and a processing circuit 150. The processing circuit 150 includes an acquiring function 150a, a display control function 150b, a training function 150c, a processing function 150d, a generating function 150e, and an accepting function 150f.

In the embodiment, respective processing functions performed in the acquiring function 150a, the display control function 150b, the training function 150c, the processing function 150d, the generating function 150e, and the accepting function 150f are stored in the memory 132 in a form of computer-executable program. The processing circuit 150 is a processor that implements functions corresponding to the respective programs by reading and executing a program from the memory 132. In other words, the processing circuit 150 that has read the respective programs is to have the respective functions indicated in the processing circuit 150.

In FIG. 1, it is explained that the processing functions performed in the acquiring function 150a, the display control function 150b, the training function 150c, the processing function 150d, the generating function 150e, and the accepting function 150f are implemented by a single unit of the processing circuit 150, but the processing circuit 150 may be constituted of plural independent processors combined, and the functions may be implemented by the respective processors executing the programs. In other words, the respective functions described above may be configured as programs, and a single unit of the processing circuit 150 may be configured to execute the respective programs. As another example, it may be configured such that a specific function is implemented by an independent dedicated program executing circuit. In FIG. 1, the acquiring function 150a, the display control function 150b, the training function 150c, the processing function 150d, the generating function 150e, and the accepting function 150f are one example of an acquiring unit, a display control unit, a training unit, a processing unit, a generating unit, and an accepting unit, respectively. Moreover, the display 135 is one example of a display unit.

A term "processor" used in the above explanation signifies a circuit, such as a central processing unit (CPU), a graphical processing unit (GPU), an application specific integrated circuit (ASIC), a programmable logic device (for example, simple programmable logic device (SPLD), complex programmable logic device (CPLD)), and a field programmable gate array (FPGA). The processor implements a function by reading and executing a program stored in the memory 132.

Moreover, instead of storing a program in the memory 132, it may be configured to directly install a program in a circuit of the processor. In this case, the processor reads and executes the program installed in the circuit, to implement the function.

The processing circuit 150 acquires various kinds of information from the medical image diagnosis apparatus by using the acquiring function 150a. The processing circuit 150 controls generation, display, and the like of an image by the display control function 150b. As one example, the processing circuit 150 causes the display 135 to display various kinds of generated images by using the display control function 150b. In addition, the processing circuit 150 may perform overall control of the medical image diagnosis apparatus in which a display control device 130 acquires data by the display control function 150b.

The processing circuit 150 generates a trained model by performing machine learning by the training function 150c. Moreover, the processing circuit 150 generates information relating to application of the trained model and the credibility (confidence level) of an output of the trained model. Details of the training function 150c, the processing function 150d, and the generating function 150e will be described later.

Moreover, in addition to this, the processing circuit 150 may generate an image based on data that is acquired from the medical image diagnosis apparatus by the generating function 150e.

The processing circuit 150 accepts various kinds of processing from a user by the accepting function 150f, for example, through the input device 134.

The memory 132 stores data acquired from the medical image diagnosis apparatus, image data generated by the processing circuit 150 including the generating function 150e, and the like. For example, the memory 132 is, for example, a semiconductor memory device, such as a random access memory (RAM) and a flash memory, a hard disk, an optical disk, or the like.

The input device 134 accepts various kinds of instructions or information input from an operator. The input device 134 is, for example, a pointing device, such as a mouse and a trackball, a selecting device, such as a mode switching switch, or an input device, such as a keyboard. The display 135 displays a graphical user interface (GUI) to accept an input of an imaging condition, an image generated by the processing circuit 150 including the generating function 150e and the like. The display 135 is, for example, a display device, such as a liquid crystal display unit.

FIG. 2 illustrates the PET apparatus 200 as an example of the medical image diagnosis apparatus equipped with the medical image processing apparatus 100. The PET apparatus 200 includes a gantry 50 and the medical image processing apparatus 100.

The gantry 50 includes a detector 51, a timing-information acquiring circuit 102, a table 103, a bed 104, and a bed driving unit 105.

The detector 51 is a detector that detects radiation by detecting a scintillation light (fluorescence) that is a light re-emitted when a substance that has become an excited state when an annihilation gamma ray emitted from a positron of the patient P and a light emitting body (scintillator) interact with each other transitions again to the ground state. The detector 51 detects energy information of radiation of the annihilation gamma ray emitted from a positron in the subject P. The detector 51 is arranged at plural positions so as to surround the subject P in a ring shape, and is constituted of, for example, plural detector blocks.

An example of a specific configuration of the detector 51 is a photo counting, or anger detector, and includes, for example, a scintillator, a light detecting device, and a light guide. That is, respective pixels included in the detector 51 have a scintillator, and a light detecting device that detects a generated scintillation light.

The scintillator converts an incident annihilation gamma ray that has been emitted from a positron in the subject P into a scintillation light (scintillation photon, optical photon), to output. The scintillator is formed by a scintillator crystal suitable for TOF measurement and energy measurement, such as lanthanum bromide (LaBr3), lutetium yttrium oxyorthosilicate (LYSO), lutetium oxyorthosilicate (LSO), lutetium gadolinium oxyorthosilicate (LGSO), and the like or BGO, and the like, and is arranged two-dimensionally.

As the light detecting device, for example, a silicon photomultiplier (SiPM) or a photo multiplier tube is used. The photomultiplier tube has a photocathode that receives a scintillation light and generates a photoelectron, a multistage dynode that gives an electric field to accelerate the generated photoelectron, and an anode that is a flow-out port of an electron, and multiplies the scintillation light output from the scintillator to convert into an electrical signal.

Moreover, the gantry 50 generates count information from an output signal from the detector 51 by the timing-information acquiring circuit 102, and stores the generated count information in a storage unit of the medical image processing apparatus 100. The detector 51 is divided into plural blocks, and includes the timing-information acquiring circuit 102.

The timing-information acquiring circuit 102 converts the output signal of the detector 51 into digital data, and generates count information. This count information includes a detecting position of an annihilation gamma ray, an energy value, and a detection time. For example, the timing-information acquiring circuit 102 identifies plural light detecting devices that have converted a scintillation light into an electrical signal at the same time. The timing-information acquiring circuit 102 identifies a scintillator number (P) that indicates a position of the scintillator to which the annihilation gamma ray has entered.

Moreover, the timing-information acquiring circuit 102 performs integral calculation of a strength of an electrical signal output from each light detecting device, to identify an energy value (E) of an annihilation gamma ray that has entered a detector 51. Furthermore, the timing-information acquiring circuit 102 identifies a detection time (T) at which a scintillation light caused by an annihilation gamma ray is detected by the detector 51. The detection time (T) may be an absolute time, or may be elapsed time from an imaging start point. As described, the timing-information acquiring circuit 102 generates the count information including the scintillator number (P), the energy value (E), and the detection time (T).

The timing-information acquiring circuit 102 is implemented, for example, by a central processing unit (CPU), and a graphical processing unit (GPU), or a circuit such as an application specific integrated circuit (ASIC), a programmable logic device (for example, simple programmable logic device (SPLD), or complex programmable logic device (CPLD)), and a field programmable gate array (FPGA).

The table 103 is a bed on which the subject P is laid, and is arranged on the bed 104. The bed driving unit 105 moves the table 103 under control by a bed control function of the processing circuit 150. For example, the bed driving unit 105 moves the subject P into an imaging port of the gantry 50 by moving the table 103.

The medical image processing apparatus 100 may have various kinds of functions as the PET apparatus 200 in addition to the functions explained in FIG. 1. For example, the processing circuit 150 included in the medical image processing apparatus 100 may generate simultaneous count information by using a simultaneous-count-information generating function not illustrated, based on the count information relating to the detector 51 that is acquired by the timing-information acquiring circuit 102. Moreover, the processing circuit 150 may reconstruct a PET image by the generating function 150e. Specifically, the processing circuit 150 reads a chronological list of the simultaneous count information stored in the memory 132, and may reconstruct a PET image by using the read chronological list, by using the generating function 105e.

Furthermore, the processing circuit 150 may perform overall control of the PET apparatus 200 by controlling the gantry 50 and the medical image processing apparatus 100, by a system control function not illustrated.

Subsequently, a background according to embodiments will be explained.

When performing image processing on a medical image, it is desirable that means to verify the credibility of the image processing itself are secured. For example, when image filter processing is performed on a medical image, it is desirable to check whether characteristics of the image filter processing itself do not affect the diagnostic performance.

From this perspective, for example, when a neural network is applied to a medical image, there is a case of taking an approach that after a trained model is generated by training with the sufficient amount of training data, the trained model is applied to another test data to verify adequacy, general versatility, and accuracy of a result.

However, by such a method, adequacy of a result after application of the neural network cannot be quantitatively evaluated for data other than the training data used at the time of generating the trained model and the test data used at the time of verification of the trained model. Accordingly, there is a case that a quantitative evaluation value cannot be presented to a user about credibility of a medical image after the medical image actually input in clinical practice is applied to the neural network.

Therefore, it is desirable that a function of quantitatively indicating credibility of an application result of a neural network to a medical image be performed in a medical image diagnosis apparatus.

In view of the background, the medical image processing apparatus according to an embodiment is a medical image processing apparatus that performs processing using a trained model to generate a first output medical image by subjecting a first input medical image to predetermined processing, and includes a processing unit that generates a second output medical image for a second input medical image by randomly switching ON/OFF connections of plural neurons included in the trained model.

Moreover, the medical image diagnosis apparatus according to an embodiment includes the medical image processing apparatus. Thus, the credibility of a result when a neural network is applied to a medical image can be presented quantitatively to a user. As an example, it becomes possible to help judgement of a user whether an amassment observed on an image is a false positive case or not when filter processing is performed by applying a neural network for a PET image.

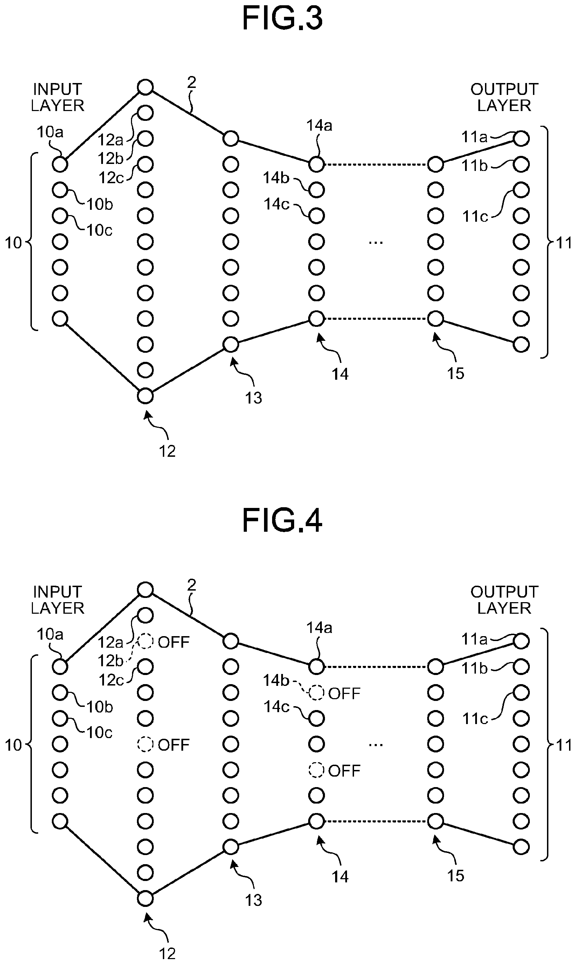

Such a configuration will be explained below by using FIG. 3 to FIG. 8.

First, an example of a trained model according to the embodiment will be explained by using FIG. 3 and FIG. 4.

In FIG. 3, a deep neural network (DNN) 2 is illustrated as an example of the neural network according to the embodiment. The DNN 2 is a neural network constituted of plural layers, and is constituted of an input layer 10, an output layer 11 that is a layer to which data is output, and their intermediate layers 12, 13, 14, and 15.

The input layer 10 denotes a layer to which data is input. Data input to the input layer 10 is a medical image or medical image data acquired from the medical image diagnosis apparatus. When the medical image diagnosis apparatus is the PET apparatus 200, data input to the input layer 10 is, for example, a PET image. Moreover, when the medical image diagnosis apparatus is an X-ray CT apparatus, a magnetic-resonance imaging apparatus, or an ultrasound diagnostic apparatus, data input to the input layer 10 is, for example, an X-ray CT image, a magnetic resonance image, or an ultrasound image, respectively. The input layer 10 includes multiple neurons, such as neurons 10a, 10b, and 10c.

Input data input to the input layer 10 may be a medical image, or various kinds of image data, projection data, intermediate data, or raw data in a previous stage before a medical image is generated. For example, when the medical image diagnosis apparatus is the PET apparatus 200, input data input to the input layer 10 may be a PET image, or various kinds of data before reconstruction of the PET image, for example, chronological data of simultaneous count information.

The output layer 11 denotes a layer to which data is output. Data output to the output layer 11 is, for example, a medical image or medical image data. Moreover, similarly to the input layer 10, the output layer 11 also have multiple neurons, such as neurons 11a, 11b, and 11c.

When the purpose of training is to perform denoise processing, data output to the output layer 11 is a medical image or medical image data having an improved image quality compared to data input to the input layer 10, subjected to, for example, denoise processing. For example, when input data input to the input layer 10 is a PET image, data output to the output layer 11 is a PET image or PET image data having an improved image quality compared to the data input to the input layer 1, subjected to the denoise processing. Furthermore, for example, when input data input to the input layer 10 is an X-ray CT image/X-ray CT data, a magnetic resonance image/magnetic resonance data, or an ultrasound image/ultrasound data, data output to the output layer 11 is an X-ray CT image/X-ray CT data, a magnetic resonance image/magnetic resonance data having an improved image quality compared to the data input to the input layer 10, subjected to the denoise processing, respectively.

Moreover, similarly to the case in which input data input to the input layer 10, data output to the output layer 11 may be a medical image, or various kinds of projection data, intermediate data, or raw data in a previous stage before generation of a medical image.

When the DNN 2 is a convolutional neural network (CNN), data input to the input layer 10 is data expressed in two-dimensional array in a size of, for example, 32×32 or the like, and data output from the output layer 11 is data expressed in two-dimensional array in a size of, for example, 32x32, and data output from the output layer 11 is data expressed in a two-dimensional array in a size of, for example, 32×32 or the like. The size of data input to the input layer 1 and the size of data output from the output layer 11 may be same, or may be different.

The DNN 2 has the intermediate layers 12, 13, 14, and 15 that hold an intermediate calculation result between the input layer 10 and the output layer 11. The intermediate layers 12, 13, 14, and 15 are also called hidden layers. The respective intermediate layers have plural neurons. For example, the intermediate layer 12 has neurons 12a, 12b, and 12c, and the intermediate layer 14 has neurons 14a, 14b, and 14c. The intermediate layer is connected to previous and subsequent layers, and an output result of a layer of a previous stage is input to a layer of a subsequent stage. For example, an output result of the input layer 10 is output to the intermediate layer 12, an output result of the intermediate layer 12 is input to the intermediate layer 13, an output result of the intermediate layer 13 is output to the intermediate layer 14, and an output result of the intermediate layer 15 is output to the output layer 11. When the DNN 2 is a CNN, the respective intermediate layers 12, 13, 14, and 15 are, for example, constituted of layers having respective unique functions, such as a pooling layer, a convolution layer, and a fully-connected layer, and by performing a predetermined calculation unique to each layer, calculation is performed based on an output result of a previous layer, and a result of the calculation is input to a subsequent layer.

Subsequently, generation of the trained model according to the embodiment, that is a training step, will be explained. The processing circuit 150 generates a trained model, for example, by performing machine learning with respect to the DNN 2 by the training function 150c. Performing machine learning means determining weights in the DNN 2, which is the neural network constituted of the input layer 10, the intermediate layers 12, 13, 14, and 15, and the output layer 11, and specifically, it means determining a set of coefficient characterizing a connection between the input layer 10 and the intermediate layer 12, a set of coefficient characterizing a connection between the intermediate layer 12 and the intermediate layer 13, ..., and a set of coefficient characterizing a connection between the intermediate layer 15 and the output layer 11. The processing circuit 150 determines these sets of coefficient by the error backpropagation method by the training function 150c.

By the training function 150c, the processing circuit 150 performs machine learning based on training data, which is supervised data constituted of data input to the input layer 10 and data output to the output layer 11, determines weights among respective layers, and generates a trained model in which weights are determined.

In deep learning, an auto encoder can be used, and in this case, data necessary in the machine learning is not necessarily supervised data.

The processing circuit 150 may generate a trained model by training the DNN 2, for example, by dropout training in which connections of plural neurons included in the trained model are randomly switched ON/OFF by the training function 150c. For example, as illustrated in FIG. 4, the processing circuit 150 generates the trained model by performing training, switching OFF the neurons 12b and 14b according to a random number at one time, and by performing training, switching OFF the neurons 12c and 14a at another time, by the training function 150c.

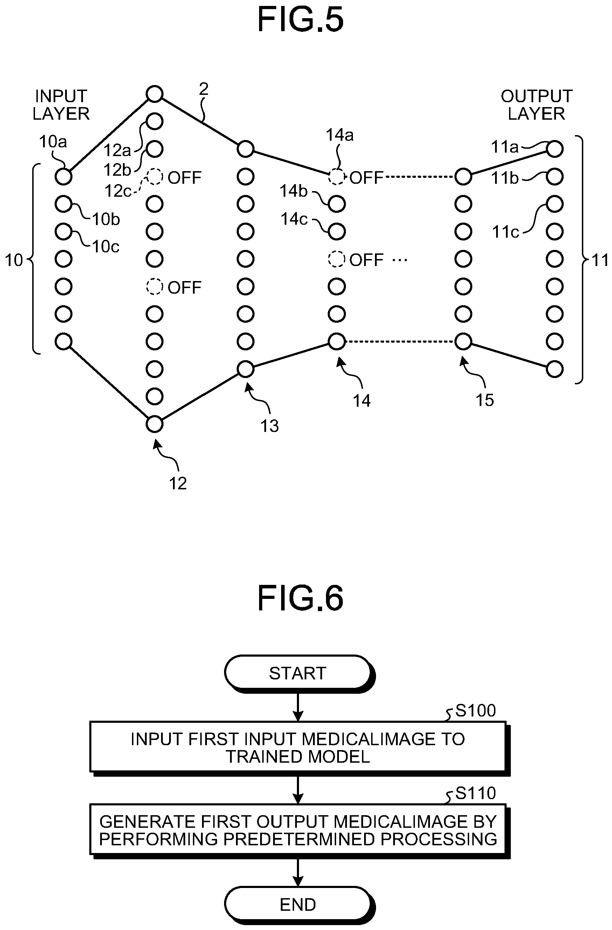

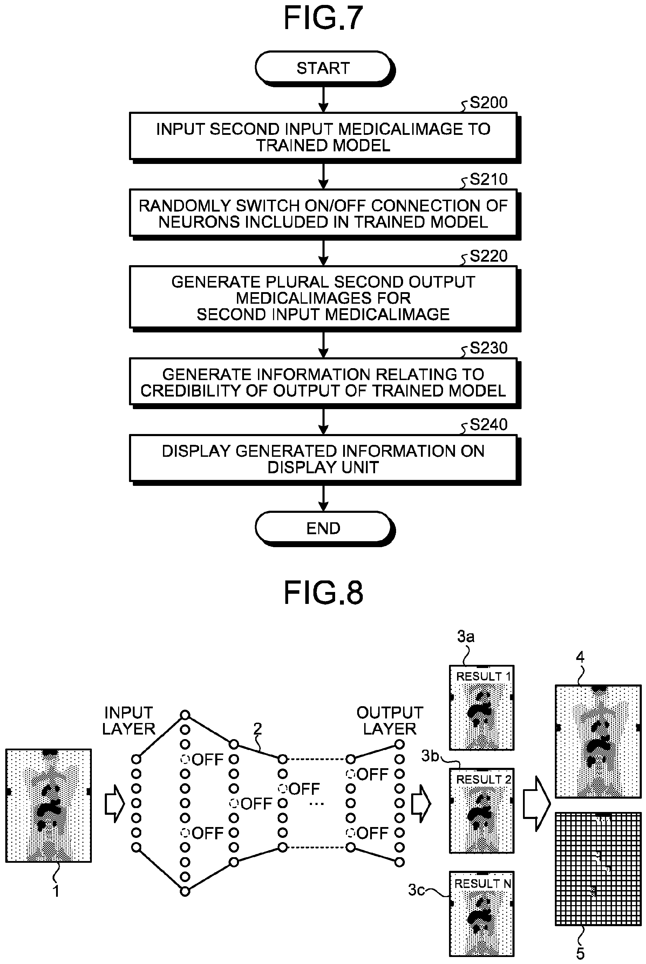

Subsequently, processing when the trained model according to the embodiment is applied to a medical image will be explained by using FIGS. 6 to 8. FIG. 6 illustrates processing when the trained model according to the embodiment is applied simply to a medical image, and FIGS. 7 and 8 illustrate processing when calculation of credibility when the trained model according to the embodiment is applied to a medical image is performed.

First, a case of applying the trained model according to the embodiment simply to a medical image will explained, and first, at step S100, the processing circuit 150 input the first input medical image, which is, for example, a clinical image, to the trained model by the processing function 150d. For example, the processing circuit 150 inputs the first input medical image, which is a clinical image, to the input layer 10 of the DNN 2, which is the trained model, by the processing function 150d. Subsequently, at step S110, the processing circuit 150 acquires data output from the output layer 11 of the DNN 2, which is the trained model, as the first input medical image by the processing function 150d. The first input medical image is a medical image subjected to predetermined processing, such as denoise processing. Thus, the processing circuit 150 generates the first output medical image that has been subjected to predetermined processing, such as denoise processing, by the processing function 150d. Moreover, the trained model according to the embodiment generates the first output medical image by performing predetermined processing with respect to the first input medical image. As necessary, the processing circuit 150 may display the acquired first output medical image on the display 135 by the display control function 150b.

Subsequently, processing of calculating credibility when the trained model is applied to a medical image will be explained by using FIG. 7 and FIG. 8.

First, as illustrated in FIG. 7, at step S200, the processing circuit 150 inputs a second input medical image 1 illustrated in FIG. 8 in the DNN 2, which is the trained model, by the processing function 150d. An expression of second input medical image is used in step S200 in FIG. 7 to distinguish from the first input medical image in FIG. 6. It is based on an intension to describe clearly that processing of calculating credibility of application of the neural network illustrated in FIG. 7 and processing of applying the neural network illustrated in FIG. 6 are separate processing. It is not excluded from the embodiment that the second input medical image is the same image as the first input medical image.

As indicated at step S210, the processing circuit 150 randomly switches ON/OFF connections between plural neurons included in the trained model when applying the trained model to the second input medical image by the processing function 150d. Thus, the processing circuit 150 generates plural pieces of second output medical images 3a, 3b, and 2c for the second input medical image by the processing function 150d as indicated at step S220.

The dropout processing is separate processing from the dropout processing at the training explained previously, but explaining this processing using FIG. 4 and FIG. 5 again, for example, as illustrated in FIG. 4, the processing circuit 150 inputs the input medical image 1 to the input layer 10 after switching OFF the neurons 12b and 14b in the DNN 2 according to a random number, and acquires an output result from the output layer 11 as the second output medical image 3a by the processing function 150d.

Moreover, for example, as illustrated in FIG. 5, the processing circuit 150 inputs the input medical image 1 to the input layer 10 after switching OFF the neurons 12c and 14a in the DNN 2 according to a random number, and acquires an output result from the output layer 11 as the second output medical image 3b, by the processing function 150d. Similarly, the processing circuit 150 inputs the input medical image 1 to the input layer 10 after switching OFF some neurons in the DNN 2 according to a random number, and acquires an output result from the output layer 11 as the second output medical image 3c, by the processing function 150d.

As for these second output medical images 3a, 3b, and 3c, since neurons to be switched ON/OFF randomly according to a random number are different although the input medical image 1 input to the input layer 10 is common, output medical images obtained are different from one another. Thus, the medical image processing apparatus according to the embodiment can acquire plural inferences from a single piece of the input medical image 1 by using the dropout processing.

Subsequently, at step S230, the processing circuit 150 generates information relating to the credibility of an output of the trained model by the generating function 150e based on plural pieces of the second output medical images generated at step S220.

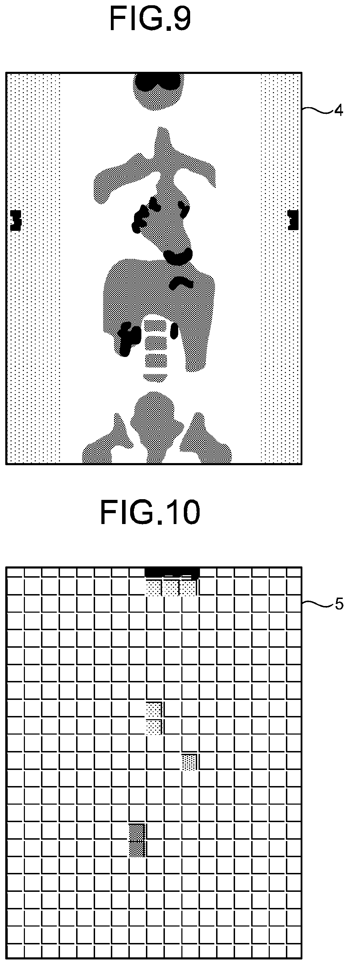

First, the processing circuit 150 generates a third output image by combining plural pieces of the second output medical images generated at step S220 by the generating function 150e. For example, as illustrated in FIGS. 8 and 9, the processing circuit 150 performs averaging for each pixel with respect to plural pieces of the second output medical images 3a, 3b, and 3c generated at step S220, and acquires a representative image that is obtained by performing the averaging as a third output medical image 4.

Moreover, the processing circuit 150 generates a fourth output medical image that is an image indicating a magnitude of variation of plural pieces of the second output medical images generated at step S200 by the generating function 150e. For example, as illustrated in FIG. 8 and FIG. 10, the processing circuit 150 calculates, for example, a standard deviation for plural pieces of the second output medical images 3a, 3b, and 3c generated at step S220, and generates an image indicating the calculated standard deviation as a fourth output medical image 5 that is an image showing a magnitude of variation of the plural pieces of the second output medical images. The fourth output medical image 5, which is an image showing the magnitude of variation of the plural pieces of the second output medical images indicates how much degree an output result is stable with respect to a small variation of the DNN 2, and in other words, it is one example of information indicating the credibility of an output of the DNN 2, which is the trained model.

Subsequently, at step S240, the processing circuit 150 causes the display 135 to display the third output medical image 4 and the information relating to the credibility of an output of the trained model by the display control function 150b. As an example, the processing circuit 150 causes the display 135 to display the third output medical image 4, which is the average image of the plural pieces of the second output medical images, and causes the display 135 to display the fourth output medical image 5, which is the image indicating the standard deviation of the plural pieces of the second output medical images, as the information indicating the credibility of an output of the DNN 2, which is the trained model, by the display control function 150b. Thus, a user can understand the credibility of an output of the DNN 2, which is the trained model, intuitively.

### Second Embodiment

In the first embodiment, a case in which plural pieces of the second output medical images are generated by performing the dropout processing in the course of processing of calculating the credibility when a trained model is applied to a medical image, and the third output medical image 4, which is their average image, is displayed as a representative image on the display 135 together with a credibility image, which is information indicating the credibility of an output of the trained model has been explained. However, embodiments are not limited thereto, and the processing circuit 150 may use a normal output medical image for which the dropout processing is not performed as a representative image to be displayed on the display 135, not an image obtained by averaging plural pieces of the second output medical images subjected to the dropout processing.

That is, in a second embodiment, the processing circuit 150 inputs the second input medical image to the trained model as the first input medical image at step S100 in FIG. 6 according to normal processing in which the dropout processing is not performed as illustrated in FIG. 6 by the processing function 150d, and handles the first output medical image generated at step S110 as the third output medical image being a representative image. On the other hand, in calculation of the information indicating the credibility of an output of the trained model, similarly to the first embodiment, the processing explained in FIG. 7 using the dropout processing is performed, and generates the fourth output medical image 5, which is an image indicating a standard deviation of plural pieces of the second output images, at step S230. Subsequently, the processing circuit 150 causes the display 135 to display the first output medical image generated at step S110 as a representative image, and the fourth output medical image 5, which is the image indicating the standard deviation, generated at step S230 as the information indicating the credibility of an output of the trained model, by the display control function 150b.

The first embodiment and the second embodiment have a point in common in displaying the information indicating the credibility of an output of the trained model, but in the second embodiment, a representative image to be displayed is an image not affected by a random number by the dropout processing.

### Third Embodiment

In the first embodiment, a case in which an image indicating a standard deviation is displayed to a user as the information indicating the credibility of an output of the trained model has been explained. However, embodiments are not limited thereto, and for example, an input of a region of interest may be accepted from a user, and information indicating the credibility of an output of the trained model may be calculated for the accepted region of interest.

In a third embodiment, first, the processing circuit 150 causes the display 135 to display the first input medical image or the first output medical image, which is an output result acquired by inputting the first input medical image to the trained model, by the display control function 150b. Subsequently, the processing circuit 150 accepts an input of a region of interest (ROI) from a user through the input device 134. Subsequently, the processing circuit 150 generates information relating to the credibility of an output of the trained model based on the region of interest by the generating function 150e.

For example, the processing circuit 150 generates the fourth output medical image, which is, for example, an image indicating a standard deviation of plural pieces of the second output medical images by performing the processing similar to that explained in the first embodiment for the region of interest by the generating function 150e. Subsequently, the processing circuit 150 calculates a value of the credibility of an output of the trained model in the region of interest specified by the user, for example, by averaging values of the fourth output medical image in the region of interest. The processing circuit 150 causes the display 135 to display the calculated value of the credibility by the display control function 150b. Thus, the credibility of an output of the trained model can be calculated for the region of interest specified by the user.

### Fourth Embodiment

In a fourth embodiment, a case in which weights in superposition are calculated based on the information indicating the credibility of an output of the trained model that has been calculated in the first embodiment, and a composite image is generated based on the calculated weights will be explained.

As an example, the processing circuit 150 calculates weights in superposition when generating a composite image based on the fourth output medical image that has been generated in the first embodiment, which is the information indicating the credibility of an output of the trained model for each pixel by the generating function 150e. For example, when the information indicating the credibility of a output of the trained model is a standard deviation image, it is regarded that the larger the standard deviation is, the lower the credibility of an output medical image of the trained model is, and therefore, it is preferable that the weight of superposition when generating a composite image be light, and inversely, it is regarded that the smaller the standard deviation is, the higher the credibility is, and therefore, it is preferable that the weight in superposition when generating a composite image be heavy. Accordingly, the processing circuit 150 calculates a weight in superposition of an output medical image when generating a composite image such that a weight in superposition of a pixel having a large value of the fourth output medical image that has been generated in the first embodiment is light compared to a weight in superposition of a pixel having a small value of the fourth output medical image, by the generating function 150e.

Subsequently, the processing circuit generates a composite image based on the calculated weight in superposition by the generating function 150e. As an example, the processing circuit 150 generates a composite medical image by superimposing the first input medical image and the first output medical image based on the calculated weight in superposition by the generating function 150e. For example, the processing circuit 150 generates a composite medical image by adding the first output medical image of the calculated weight in superposition of the output medical image to the first input medical image being an original image, by the generating function 150e. Thus, the original image and an image being an output result of the trained model can be combined appropriately, and the image quality is improved.

Embodiments are not limited thereto and, for example, the processing circuit 150 may generate a composite image by superimposing plural pieces of the second output medical images based on the calculated weight in superposition by the generating function 150e.

### Other Embodiments

In the above embodiments, a case in which the processing circuit 150 generates plural pieces of the second output medical images by using the dropout processing by the processing function 150d, and generates information relating to the credibility of an output of the trained model based on the generated plural pieces of the second output medical images has been explained. However, embodiments are not limited thereto, and in the embodiments, and ensemble inference using plural kinds of neural networks and the like may be performed instead of the dropout processing in an embodiment. That is, by performing the ensemble inference by using plural kinds of neural networks and the like, to calculate variations by respective methods, information indicating the credibility of an output of the trained model can be acquired.

In other words, the medical image processing apparatus according to the embodiment is a medical image processing apparatus that performs processing using plural trained models to generate the first output medical image by performing predetermined processing with respect to the first input medical image, and the processing circuit 150 generates plural pieces of the second output medical images for the second input medical image based on the plural trained models. That is, the processing circuit 150 generates plural pieces of the second output medical images with respect to the second input medical image, which is a single input medical image. Moreover, for example, the processing circuit 150 generates a representative image by averaging the generated plural pieces of the second output medical images by the generating function 150e. Thus, the credibility can be increased compared to a single neural network. Furthermore, the processing circuit 150 can generate the fourth output medical image, which is an image indicating a magnitude of variation of plural pieces of the second output medical images, based on the generated plural pieces of the second output medical images, by the generating function 150e similarly. Thus, the information relating to the credibility of an output of the trained model can be generated.

Moreover, in the above embodiments, a case in which the processing performed by the neural network according to the trained model is denoise processing has been explained. That is, a case in which input data input to the input layer 10 of the DNN 2 by the processing circuit 150 is medical image data/medical image including noises, and output data output to the output layer 11 of the DNN 2 by the processing circuit 150 is medical image data/medical image from which noises are removed has been explained. However, embodiments are not limited thereto. For example, processing performed by the neural network according to the trained model may be other processing, such as segmentation processing and lesion extraction processing.

Furthermore, in the embodiments, a case in which the dropout training is performed when generating the trained model has been explained. However, embodiments are not limited thereto, and the dropout training is not required to be performed at generating a trained model.

Moreover, in the embodiments, a case in which a standard deviation image is used as an image indicating a magnitude of variation of the second output medical images has been explained, but a quantity indicating the magnitude of variation is not limited to standard deviation, and it may be other quantities, such as distribution and difference between a maximum value and a minimum value.

According to at least one of the embodiments explained above, the image quality can be improved.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the scope of the invention as defined by the appended claims.

## Claims

1. A medical image processing apparatus (100) configured to perform processing using a trained model to generate a first output medical image by subjecting a first input medical image to predetermined processing, the apparatus comprising
a processing circuit (150) configured to generate a plurality of second output medical images for a second input medical image by randomly switching ON/OFF a connection of a plurality of neurons included in the trained model.

2. The medical image processing apparatus (100) according to claim 1, wherein
the processing circuit (150) is configured to generate information relating to credibility of an output of the trained model based on the second output medical images.

3. The medical image processing apparatus (100) according to claim 2, wherein
the processing circuit (150) is configured to generate a third output medical image by combining the second output medical images.

4. The medical image processing apparatus (100) according to claim 2, wherein
the processing circuit (150) is configured to generate a fourth output medical image that is an image indicating a magnitude of variation of the second output medical images.

5. The medical image processing apparatus (100) according to claim 3, wherein
the processing circuit (150) is configured to display the third output medical image and the information on a display.

6. The medical image processing apparatus (100) according to claim 2, wherein
the processing circuit (150) is configured to accept specification of a region of interest from a user, and generate the information based on the region of interest.

7. The medical image processing apparatus (100) according to claim 3, wherein
the processing circuit (150) is configured to calculate a weight in superposition for each pixel based on the information, and generate a composite medical image by superimposing the first input medical image and the first output medical image based on the weight.

8. A medical image diagnosis apparatus comprising
a medical image processing apparatus (100) according to any of claims 1 to 7.

9. A medical image processing method performed by a medical image processing apparatus (100) configured to perform processing using a trained model to generate a first output medical image by subjecting a first input medical image to predetermined processing, the method comprising
randomly switching ON/OFF a connection of a plurality of neurons included in the trained model to generate a plurality of second output medical images for a second input medical image.

10. A program configured to cause a computer performing processing using a trained model to generate a first output medical image by subjecting a first input medical image to predetermined processing, to execute
performing processing of generating a plurality of second output medical images for a second input medical image by randomly switching ON/OFF a connection of a plurality of neurons included in the trained model.
